# Europäisches Patentamt

# European Patent Office

# Office européen des brevets

(11) Veröffentlichungsnummer: **0 560 156 A2**

## EUROPÄISCHE PATENTANMELDUNG

(21) Anmeldenummer: **93103211.4**

(22) Anmeldetag: **01.03.93**

(51) Int. Cl.5: **C12N 15/11**, C12N 15/38, C12N 15/00, A01K 67/027, //C12N5/10

(30) Priorität: **13.03.92 DE 4208107**

(43) Veröffentlichungstag der Anmeldung: **15.09.93 Patentblatt 93/37**

(84) Benannte Vertragsstaaten: **BE CH DE DK ES FR GB IT LI NL SE**

(71) Anmelder: **BAYER AG**

**D-51368 Leverkusen(DE)**

(72) Erfinder: **Springer, Wolfgang, Dr.**
**Katernberger Schulweg 31**
**D-5600 Wuppertal 1(DE)**
Erfinder: **Baumgarten, Jörg, Dr.**
**Henselweg 13**
**D-5600 Wuppertal 1(DE)**
Erfinder: **Kretschmer, Axwl, Dr.**
**Richard-Zörner-Strasse 32**
**D-5060 Bergisch Gladbach 1(DE)**
Erfinder: **Kölbl, Heinz, Dr.**
**Andreas-Gryphius-Strasse 20**
**D-5000 Köln 80(DE)**
Erfinder: **Löbberding, Antonius, Dr.**
**Am Rohm 105**
**D-5600 Wuppertal 1(DE)**
Erfinder: **Strube, Walter, Dr.**
**Am Mertenshof 44**
**D-5000 Köln 40(DE)**
Erfinder: **Thein, Peter, Prof. Dr.**
**Lindenstrasse 2**
**D-8064 Oberzeitlbach(DE)**

(54) **Pseudorabies-Virus (PRV)- Polynukleotide und ihre Verwendung zur Herstellung von virusresistenten eukaryotischen Zellen.**

(57) Die Erfindung betrifft Polynukleotide, die Pseudorabies Virus-Sequenzen, insbesondere Sequenzen aus der gII-Region von PRV, in Antisense-Orientierung enthalten, ihre Herstellung und ihre Verwendung.

EP 0 560 156 A2

Rank Xerox (UK) Business Services
(3. 10 / 3.6 / 3.3. 1)

FIG. 2

EP 0 560 156 A2

Die vorliegende Erfindung betrifft Polynukleotide, die Pseudorabies Virus (PRV)-Sequenzen, insbesondere Sequenzen aus der gII Region von PRV, in Antisense-Orientierung enthalten.

Zur Herstellung von PRV resistenten Zellen werden diese Polynukleotide in virussensitive Zellen übertragen, dadurch RNA komplementär zu viralen m-RNA's von PRV gebildet und so die Synthese von für die Virusvermehrung essentiellen Glykoproteinen oder anderen Replikationsproteinen blockiert.

PRV Resistenz in Zellen und Tieren wird auch durch Antisense Oligonukleotide erreicht, die dadurch charaktersisiert sind, daß sie Gensequenzen speziell von der gII Region von PRV enthalten.

Pseudorabies Virus (PRV) ist ein Schweineherpes Virus, das zu den alpha Herpes Viren gehört und eine große Ähnlichkeit in der Struktur zur generellen Herpes Virus-Familie aufweist. Morphologisch ähnelt es dem Varizella zoster Virus und dem equinen Herpes Virus 1. PRV führt im immunokompetenten erwachsenen Tier nur sehr selten zu Krarkheitssymtomen, in jungen Tieren verlaufen PRV-Infektionen jedoch in der Regel tödlich. PRV-Infektionen sind deshalb in der Schweinezucht von großer ökonomischer Bedeutung. Darüber hinaus ist PRV in vielen domestizierten Tieren wie Rindern, Schafen, Ziegen, Hunden und Katzen sowie in vielen Wildtieren infektiös.

Das PRV-Genom umfaßt ungefähr 145 kbp und kodiert damit für ca. 100 Polypeptide. Die virale DNA besteht aus einer "unique short" Region ($U_s$), die durch inverted-repeat-Sequenzen begrenzt wird und einer "unique long" Region ohne inverted-repeat-Sequenzen. Durch Northern Blotting konnten über 70 stark exprimierte RNA-Species nachgewiesen werden.

PRV synthetisiert verschiedene Glykoproteine, die in der viralen Hülle wie auch inder Cytoplasmamembran der infizierten Zellen vorkommen. Zusätzlich findet man PRV kodierte Glykoproteine die ins Medium freigesetzt werden. Mehrere dieser Glykoproteine sind offensichtlich über Disulfidbrücken miteinander verbunden. Mindestens 7 PRV Glykoproteine werden inzwischen auf dem Virusgenom nachgewiesen.

Die Oberflächen-Glykoproteine von Herpesviren spielen eine wichtige Rolle im Infektionsprozess, in der viralen Pathogenese und in der Wechselwirkung des Virus mit dem Immunsystem des Wirtes.

Bestimmte Proteindomänen oder sogar vollständige Glykoprotein kodierende Gensequenzen können bei verschiedenen Herpesviren konserviert sein. So konnte man zeigen, daß die Glykoprotein C und D Gene von Herpes simplex Virus (HSV) große Homologie mit den PRV G III und PRV GP 50 Genen aufweisen und das Glycoprotein gB von HSV große Homologie mit der GII Glykoprotein-Familie von PRV aufweist. Glycoprotein gB von HSV zeigt auch beträchtliche Homologie mit bestimmten Glykoproteinen von Epstein Barr Virus, Varizella zoster Virus, bovinen Mammilitis Virus und dem equinen Herpes Virus Typ 1, (G. Wittmann a. H.J. Rziha, Herpes Virus Disease of cattle horses und pigs, ed. by G. Wittmann, Developments in veterinary virology Kluwer Academie Publishers, 1989.)

Die Antisense-DNA-Technologie wurde von Izant und Weintraub beschrieben (H. Weintraub, J.G. Izant, R.M. Harland Trends in Genetics, Jan. 1985, 22-25).

Angenommen wird, daß die Genaktivität dadurch gehemmt wird, daß eine Antisense-RNA von dem integrierten Polynukleotid gebildet wird, die komplementär zu der natürlichen m-RNA eines bestimmten Gens ist und damit eine AntisenseRNA-m-RNA Duplexstruktur entsteht.

Als Folge davon wird die RNA/RNA-Duplex-Struktur sehr schnell degradiert, die nukleare Prozessierung und das Splicing der m-RNA stark verzögert oder die Translation in Protein gehemmt. Als weitere Möglichkeiten des Antisense-Hemmmechanismus werden der blockierte RNA-Transport durch die perinukleare Membran und die Blockierung der Ribsomen/RNA Wechselwirkung durch doppelsträngige RNA diskutiert (E. Uhlmann a A. Peyman Chemical Reviews 90, 544-583 (1990)).

Die Regulation der Genexpression durch Antisense-RNA wurde erstmals als natürlich vorkommender Mechanismus in Prokaryonten nachgewiesen. So wurde gezeigt, daß bei Plasmidreplikation, Incompatibilität, Osmoregulation, Transponierung und Phagenreproduktion Wechselwirkungen von Sense- und kurzen Antisense-RNA-Transkripten vorkommen (P.J. Green, O. Pines, M. Inouye Ann. Rev. Biochem. 55, 569-97-(1986)).

Natürlich vorkommende Antisense-Gene wurden auch für verschiedene eukaryotische Systeme nachgewiesen, wie z.B. für das Maus-Dihydrofolatreduktasegen, das Drosophila-Dopadecarboxylasegen, die schwere Kette des Hühner Myosin-Gens oder die Satelitten RNA vom Cucumber Mosaik Virus (K.M. Tahayama und M. Inouye Critical Reviews in Biochemistry and Molecular Biology 25, 155(1990).

Die Regulation der in vivo Genexpression durch diese Antisense-Transkripte ist jedoch noch weitgehend unklar.

Die transiente Hemmung der Synthese von Genprodukten durch künstliche Antisense-Gene wurde zunächst durch verschiedene Antisense-Oligomere gezeigt. Probleme stellen dabei die Aufnahme dieser Oligomere in die Zelle, die Nuklease stabilität und die spezifische Wechselwirkung mit der Target-m-RNA dar (E. Uhlmann a.A. Peyman Chemical Reviews 90, 544-583 (1990).

Neben der Hemmung durch Oligomere wurde die künstliche Inhibition der Genexpression durch Injektion von Antisense Genen/Transkripten in Xenopus Oocyten und Embryos sowie Drosophila Embryonen gezeigt. Durch die Integration von Antisense-Genen in das Genom von eukaryotischen Zellen wurde die stabile Hemmung der Genexpression von verschiedenen Genen wie z.B. der HSV-Thymidinkinase, der Protooncogene c-fos, c-myc, c-src sowie von ß-Actin-Genen, von HIV-Virus-Genen und auch einigen Pflanzengenen wie z.B. dem Tomaten-Polygalacturonase Gen nachgewiesen, Auch in transgenen Organismen wurde die Wirkung von Antisense-Genen gezeigt.

Die vorliegende Erfindung betrifft:

1. Antisense DNA Polynukleotide, die dadurch gekennzeichnet sind, daß sie Gensequenzen von PRV enthalten, die in Antisenseorientierung zu einer Promotorsequenz lokalisiert sind.

2. Antisense DNA Polynukleotide, die dadurch gekennzeichnet sind, daß sie Gensequenzen aus der gII-Region von PRV enthalten, die in Antisenseorientietung zu einer Promotorsequenz lokalisiert sind.

3. Antisense DNA Polynukleotide, die dadurch gekennzeichnet sind, daß sie eine 180 bp große Region entsprechend der Nukleotidsequenz 879-1058 der gII-Region von PRV enthalten, die in Antisenseorientierung zu einer Promotorsequenz lokalisiert ist.

4. Antisense RNA Polynukleotide, die dadurch gekennzeichnet sind, daß sie Gensequenzen von PRV enthalten.

5. Antisense RNA Polynukleotide, die dadurch gekennzeichnet sind, daß sie Gensequenzen aus der gII-Region von PRV enthalten.

6. Antisense RNA Polynukleotide, die dadurch gekennzeichnet sind, daß sie eine 180 bp große Region entsprechend der Nukleotidsequenz 879-1058 der gII-Region von PRV enthalten.

7. Antisense Oligonukleotide, die dadurch gekennzeichnet sind, daß sie Gensequenzen von PRV enthalten, die komplementär zu viralen RNA's von PRV sind.

8. Antisense Oligonukleotide, die dadurch gekennzeichnet sind, daß sie Gensequenzen von der gII-Region von PRV enthalten, die komplementär zur viralen gII RNA von PRV sind.

9. Antisense Oligonukleotide, die dadurch gekennzeichnet sind, daß sie Gensequenzen aus einer 180 bp großen Region (879-1058) aus dem gII-Bereich von PRV enthalten, die komplementär zur viralen gII RNA sind.

10. Expressionsvektorkonstrukte enthaltend Polynukleotide aus PRV in Antisense-Orientierung zu Promotorsequenzen.

11. Expressionsvektorkonstrukte enthaltend Polynukleotide aus der gII-Region von PRV in Antisense-Orientierung zu Promotorsequenzen.

12. Expressionsvektorkonstrukte enthaltend ein 180 bp Polynukleotid (879-1058) aus der gII-Region von PRV in Antisense-Orientierung zu Promotorsequenzen.

13. Zellen und Nachkommen dieser Zellen enthaltend Antisense Polynukleotide nach Punkten 1 bis 6.

14. Schweine, die in ihren Zellen Antisense-Polynukleotide nach Punkten 1 bis 6 und 10 bis 12 enthalten.

15. Schweine, die in ihren Zellen Antisense-Oligonukleotide nach Punkten 7 bis 9 enthalten.

16. Verfahren zur Herstellung von PRV resistenten Zellen, dadurch gekennzeichnet, daß Polynukleotide nach Punkten 1 bis 6 oder 10 bis 12 in Virus sensitive Zellen übertragen werden und RNA komplementär zur viralen RNA gebildet wird und dadurch die virale Vermehrung von PRV verhindert wird.

17. Verfahren zur Hemmung von PRV-Infektionen, dadurch gekennzeichnet, daß Antisense-Oligonukleotide nach Punkten 7 bis 9 in Tiere appliziert werden.

18. Verwendung von Polynukleotide gemäß Punkten 1 bis 6 oder 10 bis 12 zur Übertragung in Zellen, die zur Erzeugung von transgenen PRV-resistenten Tieren geeignet sind.

Die vorstehend verwendeten Begriffe haben folgende Bedeutung:

Antisense-DNA-Polynukleotide sind relativ kleine natürliche oder synthetische Nukleinsäure-Polymere, die von minimal 10-20 Nukleotidbasen bis zu mehreren tausend Nukleotidbasen enthalten können. Diese Polynukleotide werden in Zellen übertragen, die sensitiv gegenüber PRV-Viren sind. Als Transformation wird dabei das Verfahren bezeichnet, bei dem der Genotyp einer Rezipientenzelle durch Einführung von z.B. Antisense-Polynukleotiden verändert wird. Als Antisense-DNA-Polynukleotide werden solche Polynukleotide bezeichnet, die einzelsträngige oder doppelsträngige DNA-Moleküle enthalten, wobei der kodierende Strang in 3'---5'-Richtung transkribiert werden kann und dabei Antisense-RNA gebildet wird, die komplementär zur Virus-m-RNA ist. Durch die gebildete RNA/RNA-Duplex-Struktur kann keine virale Proteinsynthese mehr erfolgen. Zur Transkription von Antisense-RNA enthält das Antisense-DNA-Polynukleotid Transkriptionssignale, sogenannte Promotorsequenzen, z.B. des SV40 Promotors vor dem in Antisense-Orientierung vorliegenden PRV-Genfragment. Durch Auswahl von bestimmten Regionen aus dem PRV-Genom wie z.B. aus der gII-Region von PRV oder Teilen der gII-Region können Antisense-Polynukleotide konstruiert werden, die besonders effektiv die PRV-Virussynthese hemmen.

4

Antisense-RNA-Polynükleotide sind einzelsträngige RNA-Moleküle mit einer Länge von einigen hundert bis mehreren tausend Nukleotidbasen, die komplementär zu einer Region der viralen RNA sind und dadurch die Translation der viralen m-RNA hemmen können. Im Gegensatz zu Antisense DNA-Polynukleotiden können Antisense-RNA-Polynukleotide nicht stabil in das Genom von Rezipientenzellen integriert werden.

Antisense-Oligonukleotide sind einzelsträngige DNA-Moleküle, die von 10-20 Nukleotidbasen bis zu 100 Nukleotidbasen enthalten. Neben den natürlichen nukleasesensitiven Oligonukleotiddiestermolekülen gehören dazu nukleaseresistente Oligonukleotidderivate wie z.B. Methylphosphonat-Oligonukleotide, Phosphorothioat-Oligonukleotide, 5'Amidat-Oligonukleotide, peptide cleic acids (S.J.C. Hanveg et al., Science 258, 1481 (1992)) or other pseudooligouncleotides.. Die Sequenz dieser Oligonukleotide ist komplementär zu bestimmten Regionen der viralen RNA.

Zur Erzielung der antiviralen Wirkung müssen die Oligonukleotide über die natürlichen Aufnahmemechanismen der Zelle aufgenommen werden. Zur Erzeugung einer PRV-Resistenz im Tier müssen die Oligonukleotide mit geeigneten Methoden in das Tier appliziert werden.

Als Antisense-Vektor-Konstrukte sind eukaryotische Expressionsvektoren geeignet, die PRV-DNA in Antisenseorientierung zu Promotoren enthalten. Solche Vektoren verfügen in der Regel über Bereiche, sogenannte Replikons, die zur Vermehrung in Prokaryonten (z.B. pMB1) oder Eukaryonten (z.B. SV40) befähigen. Darüber hinaus sind Transkriptionssignale, sogenannte Promotorsequenzen, vorhanden, die die Transkription von dahinter insertierter Antisense-DNA erlauben. Solche Promotorsequenzen sind z.B. der frühe oder späte SV40-Promotor, der Thymidinkinase-Promotor oder der Metallothionin-Promotor. Mit steigender Zahl von Antisense-RNA-Transkripten steigt auch die Wahrscheinlichkeit durch höheren Export aus dem Nukleus oder stärkerer Akkumulation im Cytoplasma eine bessere Wirksamkeit der Antisense-Inhibition zu erzeugen.

Die Terminierung der Antisense-RNA-Transkripte erfolgt mit dem Polyadenylierungssignal z.B. aus SV40 oder dem bovinen Wachstumshormon um eine hochstabile m-RNA zu erzeugen. Weiterhin enthalten die Vektoren Gene zur Expression von Selektionsmarkern in Prokaryonten, z.B. Ampicillinresistenz und Eukaryonten z.B. Neomycin oder G418 um Zellen selektionieren zu können, die nach der Transfektion das Genkonstrukt für die Antisense-RNA Expression enthalten. Dieser Selektionsmarker hat sich für tierische PRV-sensitive Zellen bewährt. Andere Selektionsmarker wie z.B. Puromycin, Hygromycin oder Methotrexate in Kombination mit der Transfektion des Gens für die Dihydrofolsäure-Reduktase können ebenfalls verwendet werden.

Virussensitive Zellen sind embryonale Hühnerfibroplasten, primäre Nierenzellen von Kaninchen, Pferd, Rind, Hund, Katze, Affe, Hamster, Verozellen, BHK-Zellen, ED-Zellen und HeLazellen.

Virussensitive Zellen des Schweins sind insbesondere die Zellen des gesamten oberen und unteren Respirationstrakts, der Nasenschleimhaut und des Lungengewebes sowie regionäre Lymphknoten wie z.B. Tonsillen und Zellen des Gehirns und übrigen Nervengewebes wie auch Blutzellen und Zellen des Uterus- und Foetus-Gewebes.

Zur Herstellung der erfindungsgemäßen Antisense-DNA-Polynukleotide können beliebige Stämme von PRV eingesetzt werden. Solche werden in an sich bekannter Weise von infizierten Tieren entnommen und in an sich bekannter Weise in üblichen Vermehrungsmedien in virussensitiven Zellen vermehrt. Nach Isolierung der PRV Nukleinsäure werden mit üblichen gentechnologischen Methoden wie z.B. im Beispiel 1 beschrieben E.coli Klone hergestellt, die in einem Expressionsvektor PRV-Genfragmente in Antisense-Orientierung zu einem Eukaryontenpromotor enthalten. Diese PRV-Genfragmente werden mit Hilfe des Vektors in E.Coli vermehrt, die DNA in an sich bekannter Weise isoliert und die PRV-DNA mit dem Promotor und den Terminationssequenzen mit Hilfe von Restriktionsenzymen aus dem Vektor ausgeschnitten und isoliert oder das gesamte Vektorkontrukt zur Herstellung von virusresistenten Zellen eingesetzt.

Die chemische Synthese von Antisense-Oligonnkleotiden kann auf der Basis der publizierten Sequenz von PRV (Robbis et al., J.Virology, 61, 2692-2701, 1987) nach der Amidit-Methode von S.L. Beaucage und M.H. Caruthers, Tetrahedron Letters 22, 1859-1862(1981) durchgeführt werden.

Oligonukleotidderivate wie Methylphosphonate oder Phosphorothioate können nach publizierten Verfahren hergestellt werden (W.J. Stec et al, J.Am.Chem.Soc. 106, 6077, 1984; B.C.Froehler, Tetrahedron Letters 27, 5575, 1986; A. Jäger, J. Engels, Tetrahedron Letters 25, 1437, 1984).

Antisense-RNA-Polynukleotide können mit Hilfe von üblichen gentechnologischen Methoden hergestellt werden. PRV-Genfragmente, insbesondere der gII Region, werden dazu in Vektoren in Antisense-Orientierung zu starken RNA-Polymerase-Promotoren wie SP6, T3, T7 kloniert und Antisense-RNA mit kommerziell erhältlichen in vitro Transkriptionssystemen hergestellt.

In der vorliegenden Erfindung wird beispielhaft die Wirkung von verschiedenen Antisense-DNA-Fragmenten aus dem Bereich des gII Glykoproteingens von PRV auf die virale Genreplikation beschrieben.

Das gII Gen von PRV wurde hierzu aus einem E.coli Plasmid mit üblichen gentechnologischen Methoden in einen Prokaryonten-Eukaryonten-Vektor kloniert, so daß das gII Gen in Sense und Antisense Orientierung zu dem SV40 Promotor des Vektors vorliegt (G. Wittman a. H.J.Rziha, Herpes Virus Disease of Cattle, horses and pigs, ed, by G. Wittman, Developments in veterinary virology Kluwer Academie Publishers, 1989). Prinzipiell können für den Zweck der Antisense-RNA-Expression auch andere Promotorsequenzelemente oder Hybride von zwei verschiedenen Promotorsequenzelementen verwendet werden, die in den Zielzellen eine starke Expression der Antisense RNA erwarten lassen. Ebenso können zur Terminierung und Polyadenylierung der Antisense-RNA verschiedene Terminationssequenzen für die Optimierung von RNA-Processing, Transport und Stabilität verwendet werden. Hier werden Beispiele für die SV40 early Promotorsequenz und die SV40 small t Antigen Termination Sequenz gegeben.

Neben dem nahezu gesamten gII Gen wurden auch DNA-Fragmente in der Größe von 0,1 bis 1,5 kB in ähnlicher Weise in den pBEH Vektor kloniert Die Sense oder Antisense-Orientierung der PRV gII-Fragmente wurde durch Sequenzierung dieser Konstrukte überprüft.

Diese PRV gII-Antisense-Konstrukte wurden mit zellbiologischen Techniken in Zellen transfiziert, die sensitiv für PRV-Viren waren. Klone, die Antisensekonstrukte enthielten, wurden über die G418 Resistenz von cotransfiziertcn Neomycinresistenzkonstrukten isoliert.

Im Hemmtest mit Pseudorabies-Viren wurde überprüft, ob Klone entstanden waren, die aufgrund der exprimierten PRV gII-Antisense-RNA resistent gegen die Virusinfektionen bzw. Virusreplikation geworden waren.

Dabei wurde z.B. mit einem 180 bp langen Antisense-Konstrukt aus dem Nukleotidbereich 879 bis 1058 der gII-Region von PRV eine starke Hemmung der PRV-Virusvermehrung festgestellt.

Der Nachweis, daß diese virusresistenten Zellklone tatsächlich Antisense-DNA-Konstrukte enthielten, wurde mit einer DNA-Hybridisierungstechnik, der sog. Southern Blot Technik in Verbindung mit der Polymerase Chain Reaktion (PCR) durchgeführt. Die Expression der Antisense-RNA in den virusresistenten Zellklonen wurde mit der Northern Blot-Hybridisierungstechnik überprüft

Die vorliegende Erfindung betrifft bevorzugt Antisense DNA Polynukleotide, die dadurch gekennzeichnet sind, daß sie Gensequenzen von PRV Gensequenzen aus der gII-Region von PRV oder einer 180 bp großen Region entsprechend der Nukleotidsequenz 879-1058 der gII-Region von PRV enthalten, die in Antisense-Orientierung zu einer Promotorsequenz lokalisiert sind.

Die vorliegende Erfindung betrifft bevortugt Antisense RNA Polynukleotide, die dadurch gekennzeichnet sind, daß sie Gensequenzen von PRV, der gII-Region von PRV oder einer 180 bp großen Region entsprechend der Nukleotidsequenz 879-1058 der gII-Region von PRV in Antisense-Orientierung enthalten.

Dazu gehören auch bevorzugt Antisense-Oligonukleotide, die dadurch gekennzeichnet sind, daß sie Gensequenzen von PRV, der gII-Region von PRV oder einer 180 bp großen Region (879-1058) aus der gII-Region von PRV enthalten, die komplementär zur viralen PRV RNA sind.

Die Erfindung beinhaltet auch bevorzugt Expressionsvektorkonstrukte, die Polynukleotide aus PRV, aus der gII-Region von PRV oder einem 180 bp Polynukleotid (879-1058) aus der gII-Region von PRV enthalten in Antisense-Orientierung zur Promotorsequenzen, sowie Zellen und Nachkommen dieser Zellen, wie auch Tiere die diese Antisense-Polynukleotide oder Expressionsvektorkonstrukte enthalten.

Die Erfindung betrifft auch Verfahren zur Herstellung von PRV resistenten Zellen, dadurch gekennzeichnet, daß Antisense-Polynukleotide wie auch Expressionsvektorkonstrukte mit Antisense-Polynukleotide in virussensitive Zellen übertragen werden und RNA komplementär zur viralen RNA gebildet wird und dadurch die virale Vermehrung von PRV verhindert wird. Dazu gehören Verfahren zur Hemmung von PRV-Infektionen, dadurch gekennzeichnet, daß Antisense-Oligonukleotide in Tiere appliziert werden. Die Erfindung betrifft auch die Verwendung von Antisense-Polynukleotide zur Übetragung in Zellen, die zur Erzeugung von transgenen PRV- resistenten Tieren geeignet sind.

**Beispiele**

**Beispiel 1**

**Gentechnologische Herstellung von PRV-Antisense-DNA-Konstrukten**

**Isolierung von PRV gII DNA**

Ausgangsbasis für die Isolierung des gII Glykoprotein-Gens von PRV war der Klon PRV1A, der von der Bundesforschungsanstalt für Viruskrankheiten der Tiere, Tübingen, zur Verfügung gestellt wurde.

Aus dem E.coli Klon PRV1A wurde das rekombinante Plasmid mit der gII Sequenz präparativ isoliert. Dazu wurden 500 ml Übernachtkultur in LB-Medium mit Ampicillin (50 mg/l) bei 4 000 UpM 10 Minuten bei 40° C in einer Kühlzentrifuge abzentrilugiert und der Zellniederschiag in 10 ml Lysozymmix (9 mi 25 % Saccharose in 50 mM Tris/HCl pH 7,5 und 1 ml 10 mg/ml Lysozym) suspendiert. Die Zellen wurden mindestens 30 Minuten bei Raumtemperatur inkubiert Dann wurden 2 mi 500 mM EDTA pH 8 und 15 ml Tritonmix (5 mi Triton, 62,5 ml, 500 mM EDTA, 25 mi 1 M Tris/HCl pH 8, 33.75 ml/$H_2O$) zugegeben und solange geschüttelt, bis nur noch eine homogene Phase zu sehen war.

Die Proben wurden 30 Minuten in Eis gestellt und dann 1 Stunde bei 13000 UpM in der Kühlzentrifuge (JA 17 Rotor, Beckmann) zentrifugiert. Der Überstand wurde in Ultrazentrifugenröhrchen überführt (28 g CsCl, 30 g Nukleinsäureüberstand, 4 ml Ethidiumbromid (10 ml/ml)). Die Röhrchen wurden verschlossen und im Ti$^5$O-Rotor mindestens 14 Stunden bei 45 000 UpM und 20° C zentrifügiert. Nach der Zentrifugation wurde unter dem UV-Licht die untere Plasmid-Bande mit Hilfe einer Kanüle isoliert und das Ethidiumbromid durch mehrmalige Extraktion mit CsCl-gesättigtem Isopropylalkohol entfernt.

Das Cäsiumchlorid wurde aus der DNA-Lösung entfernt durch 3-stündige Dialyse gegen TE-Puffer bei stündlichem Pufferwechsel.

LB-Medium enthält 10g Bactotrypton, 5 g Hefeextrakt, 10g NaCl 11 $H_2O$ pH 7,5

TE-Puffer enthält 10 mM Tris/HCl pH 8,0,1 mM EDTA

## Klonierung der gII DNA in Eukaryonten-Expressionsvektoren

Das rekombinante Plasmid aus dem Klon PRV1A, das die gII Sequenz enthält wurde mit dem Restriktionssequenzen Sph I gespalten und das 2,9 kB große Sph-gII-Fragment durch präperative Agarose-Gel-Elektrophorese und Elektroelution mit dem Elektroelutor der Fa. International Biotechnologies Inc., New Haven isoliert.

Mit üblichen gentechnologischen Methoden (Maniatis et al., Molecular Cloning, Gold Spring Harbor Laboratory Press, 1989) wurde das 2,9 kb große Sph I-gII Fragment zunächst in die SpH I-Site des Vektors pUC 19 (Pharmacia, Uppsala, Schweden) kloniert. Aus einem entsprechenden pUC 19-Klon wurde das rekombinante Plasmid pUC 19 mit dem SpH I gII-Fragment isoliert und das gII-Fragment mit dem Restriktionsenzymen EcoRI und Hind III aus der Polylinker-Region von pUC 19 herausgespalten und über Agarosegelelektrophorese und Elektroelution isoliert.

Das 2,9 kb große EcoRI/Hind III-gII-Fragment wurde direkt oder nach Spaltung mit dem Restriktionsenzym Sau III A zur weiteren Klonierung in Eukaryonten-Expressionsvektoren eingesetzt.

Die Klonierung des 2,9 kb großen EcoRI/HindIII-gII-Fragmentes und der SauIII-Fragmente von PRV wurde der Eukaryonten-Expressionsvektor pBEH verwendet, der von der GBF Braunschweig zur Verfügung gestellt wurde. Die Klonierungssirategie wurde so gewählt, daß das EcoRI/HindIII gII-Fragment in Antisense-Orientierung vom SV40 early Promotor exprimiert wird. Die SauIIIa Fragmente der gII Region wurden über die BamHI Site des Polylinkers von pBEH in Sense und Antisense-Orientierung kloniert. Die DNA-Konstrukte wurden durch Elektroporation in E.coli transformiert.

Zur Elektroporation wurden zunächst elektrokompetente E.coli Zellen hergestellt. 500 ml E.coli Kulturlösung wurden bei einer OD von 0,5(550 nm) bei 4 000 g abzentrifugiert. Die Zellen wurden im gleichen Volumen eiskaltem sterilen bidest $H_2O$ gewaschen. Das Waschen wurde zweimal wiederholt. Anschließend wurden die Zellen mit 50 ml kaltem 10 %igen Glycerin/bidest $H_2O$ gewaschen, bei 5 000 UpM zentrifugiert und dann in 1-5 ml 10 %igem, Glycerin/bidest $H_2O$ aufgenommen. Die Zellen wurden bei -70° C gelagert.

Die Elektroporation (EP) wurde mit dem Gene Pulser Apparatus and Capacitans Extender der Fa. BioRad durchgeführt.

50 ml Zellen wurden auf Eis aufgetaut, in eine EP-Küvette 0,2 cm überführt und 10-50 $\mu$g DNA in 5-10 $\mu$l bidest $H_2O$ zugesetzt.

Die EP Bedingungen waren:

Kapazität: 250 uF

Spannung: 2,5 kv

Widerstand: 200 ohm

G. Pulser: 25 uF

Elektrodenabstand: 0,2cm

Der Impuls sollte nach der EP bei 4-5 msec liegen. Nach dem Pulsen wurde sofort 1 mi SOC-Medium (2 % Bacto Trypton, 0,5 % Bacto Yeanst Extrakt 10 mM NaCl, 2,5 mM KCl, 10 mM $MgCl_2$, 10 mMMg $SO_4$,20 mM Glucose) zugegeben und der Küvetteninhalt in ein steriles Röhrchen überführt. Nach 1 Stunde Zwischenzüchtung bei 37° C wurde ausplattiert. E.coli Klone, die die Ampicillinresistenz des pBEH-Vektors exprimieren, wurden molekularbiologisch charakterisiert

**Molekulare Charakterisierung der PRV-Expressionskonstrukte in E.coli**

Zur molekularbiologischen Chaiakterisierung der pBEH enthaltenden Klone wurden die Plasmide nach einem üblichen analytischen Nükleinsäureisolierungsverfahren isoliert.

Durch Agarosegelelektrophorese wurden solche Klone identifiziert, die in den pBEH Vektor das EcoRI/HindIII gII-Fragment oder SaulIIA gII Fragmente insertiert hatten.

Zum Nachweis der Sense oder Antisense-Orientierung der SauIII A gII-Fragmente im pBEH Vektor wurden die SauIII A gII-Fragmente unter Verwendung von SV40 Primern nach der Methode von Sanger mit einem Sequenase-Kit der Firma USB, Cleveland, Ohio, ansequenziert und die Orientierung durch Vergleich mit der bereits publizierten Sequenz der gII-Region bestimmt.

In der folgenden Tabelle 1 sind die in der gII-Sequenz vorhandenen SauII A-Fragmente aufgelistet. Beispielhaft für die verschiedenen klonierten und getesteten Sense- und Antisense-PRV-Fragmente werden 5 Antisense-Konstrukte aufgeführt, die durch Insertgröße, Nukieotidbereich und Antisense-Orientierung charakterisiert wurden.

In Abbildung Fig. 1 ist ein Ausschnitt aus der Restriktionskarte von PRV mit der gII-Region von PRV dargestellt und die Lokalisierung von beispielhaft aufgefühiten Antisense-Konstrukten eingezeichnet. In Abbildung Fig. 2 ist eine Restriktionskarte des Vektorkonstruktes SPN 345-1, aufgeführt, das aus dem Vektor pBEH und einem 180 bp großen Sau III A-Fragemnt (Nukleotidbereich 879-1058) besteht, das in Antisenseorientierung zu den SV40 Promotor des Vektors integriert ist. In Abbildung Fig. 3 ist die Nukleotidsequenz des gII-Fragmentes aus dem Klon SPN 345.1 angegeben.

## TABELLE 1

| Klonbezeichnung | Insertgröße | Bereich gII-Region | Orientierung |
|---|---|---|---|
| 336-5 | 250 bp | 1417-1653 | Antisense |
| 345-1 | 180 bp | 870-1058 | Antisense |
| 345-14 (351-4) | 510 bp | 1662-2172 | Antisense |
| 345-39 | 600 bp | 1059-1662 | Antisense |
| 891-1 | 2900 bp | -883 bis 2028 | Antisense |

### SauIIIA Fragmente auf PRVG2222 und PRV gII

| Bereich | Fragmentgröße | Bereich | Fragmentgröße |
|---|---|---|---|
| 0 - 217 | 217 | 1417-1663 | 246 |
| 217- 527 | 310 | 1663-2173 | 510 |
| 527- 728 | 201 | 2173-2329 | 159 |
| 728- 881 | 153 | 2329-2443 | 114 |
| 881- 893 | 12 | 2443-2611 | 168 |
| 879-1058 | 180 | 2611-2845 | 234 |
| 1059-1417 | 358 | 2845-3048 | 203 |
| 1059-1663 | 604 | | |

### SPHI Fragmente auf PRVG222

| Bereich | Fragmentgröße |
|---|---|
| 0-2033 | 2033 |
| 2033-3048 | 1015 |

**Beispiel 2**

**Transfektion und Klonierung von Verozellen mit PRV Antisense-RNA Expressionsvektoren**

Als Beispiele von transfizierten tierischen Zellen, die Virusresistenz durch endogen exprimierte Antisense-RNA gegen PRV autweisen, wurden adhärent wachsende Verozellen isgewählt. Die Zellinie wurde von der American Type Culture Collection (ATCC), 12301 Parklawn Drive, Rockville, MD 20852-1776, USA erhalten.

Die Zellinie wurde in Medium 199 (Firma Flow) mit 5 % FCS (foetales Kälberserum) und 7,5 ml Penicillin/Streptomycin (10 mg/ml) 5 ml L-Glutamin und 5 ml nicht essentielle Aminosäuren (Eagle (1959) Science 1343) kultiviert. Die Vektortransfektion erfolgte durch die Calciumphosphat-Methode, die beste Erfolge zeigte:

Caiciumphosphonat Methode:

Variiert nach Wingler et. al., 1977, Cell 11,223

Die Transfektion wird mit Ca-Präzipitaten der DNA durchgeführt. Variierte Mengen von 0,05 bis 20 $\mu$g DNA wurden in 0,25 ml 250 mM $CaCl_2$ gelöst und langsam zu 0,25 ml 2 x HEBS-Puffer pipettiert. Die Transfektion erfolgte durch Zugabe des gesamten Präzipitat-Ansatzes in 10 ml Zell-Kultur. 15 Stunden später wurde das Medium abgesaugt. Danach wurden die Zellen mit DMSO geschockt.

Für eine 10 ml Kultur wurden 3 ml einer 25 %igen DMSO-Lösung in sterilem PBS hinzugegeben. Nach 4 Min. wurde abgesaugt und mit 5 ml PBS gewaschen. Anschließend wurden die Zellen in 10 ml DMEM Medium aufgenommen.

HEBS-Puffer enthält 50 mMol Hepes (N-2-Hydroxyethylpiperazine-N'-2-ethan, 280 mMol NaCl Sulfonsäurenatriumsalz)

PBS (Phosphate buffered säline) enthält in g/Liter: KCl 2; $KH_2PO_4$ 2; NaCl 80; $Na_2HPO_4 \cdot 7H_2O$ 21,6;

DMEM Medium (Dulbecco Modifikation Eagle Medium) enthält in mg/Liter L-Arginin 84; L-Cystin 56,78; L-Glutamin 584; Glycin 30; L-Histidin 42; L-Isoleucin 104,8; L-Leucin 104,8, L-Lysin 146,2, L-Methionin 30, L-Phenyl-alanin 66; L-Serin 42; L-Threonin 95,2; L-Tryptophan 16; L-Tyrosin 89,5; L-Valin 93,6; D-Ca-Pantothenat 4; Cholinchlorid 4; Folsäure 4; Inosit 7; Nicotinamid 4; Pyridoxal HCl 4; Riboflavin 0,4; Thiamin 4; $CaCl_2 \cdot 2H_2O$ 264,9; $Fe(NO_3)_3 \cdot 9H_2O$ 0,1; KCl 400; $MgSO_4 \cdot 7H_2O$ 200; NaCl 6400; $NaHCO_3$ 3700; $NaH_2PO_4 \cdot 2H_2O$ 141,3; D-Glucose 4500; Phenolrot 15, Natriumpyruvat 15;

## Selektion von Transtektanten

48 Stunden nach dem DMSO-Schock wurden die Zellen trypsiniert und in Selektionsmedium (Nährlösung für Verozellen + 0,74 mg/ml Geneticin G418) resuspendiert. Die Zellen einer Kulturplatte wurden dabei auf insgesamt 5 Platten verteilt und mit Selektionsmedium auf 10 ml Volumen aufgefüllt.

Die Zellen wurden anschließend bei 37°C im $CO_2$-Brutschrank weiterinkubiert, wobei anfangs alle 3 Tage Mediumwechsel vorgenommen wurde. Der Mediumwechsel wurde solange fortgesetzt bis alle nicht transfizierten Zellen abgestorben waren und die transfizierten Zellen von Kolonien vorsichtig mit einer Eppendorfpipette abgesaugt. Die Zellen wurden anschließend in 0,2 ml Selektionsmedium überführt und solange expandiert bis genügend Zellen zur Testung der Virusresistenz gewachsen waren.

## Beispiel 3

## Nachweis der Integration von Antisense-PRV-Konstrukten im Genom von eukaryotischen Zellen

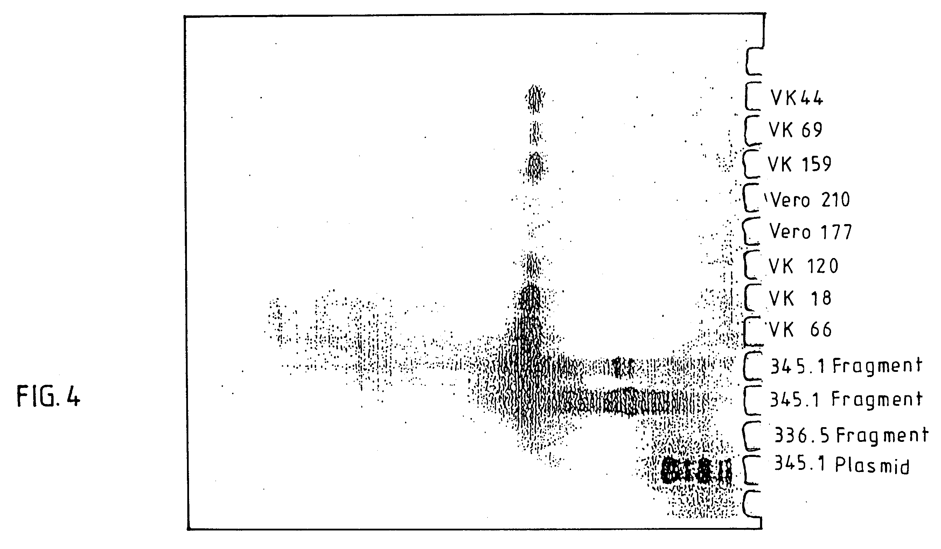

Die G418 resistenten Veroklone (in der Abbildung Fig. 4 mit VK18, VK44, VK66, VK69, VK120 und VK159 bezeichnet) sowie die nicht transfizierten Verozellinien (in Abbildung Fig. 4 mit V177 und V210) bezeichnet wurden mit der Southern Blot Technik (L.G. Davis et al., Basis Methods in Molecular Biology, Elsevier, New York (1986)) in Kombination mit der Polymerasekettenreaktion (PCR) (PCR Technology Priciples and Application for DNA Amplification, Ed. Henry A. Erlich, M. Stockton Press, New York (1989))- auf Integration der PRV gII Antisense-DNA-Konstrukte analysiert. Dazu wurden zunächst 50 ml Zellkulturen von den verschiedenen Veroklonen angezüchtet. Die Zellen wurden bei 2 000UpM in einer Beckmann Kühlzentrituge mit einem JA 17 Rotor abzentrifugiert Das Zellpellet wurde anschließend in 3 ml Tris/HCl-Puffer pH 8 (0,025 M Tris/HCl 0,025 M EDTA, 0,3 M Saccharose) resuspendiert und 30 Minuten bei Raumtemperatur inkubiert. Nach Zugabe von 300 $\mu$l 0,5M EDTA pH 8,0 und 30 $\mu$l 20 %iges Natriumdode-cylsulfat lysieren die Zellen. Zur Degradation der RNA wurde das Lysat mit 70 $\mu$l RNase (1 mg/ml) versetzt und 30 Minuten bei 37°C inkubiert. Das Lysat wurde mit TE-Puffer auf 20 ml verdünnt und anschließend zweimal mit dem gleichen Volumen Tris gesättigten Phenol behandelt. Die wässrige obere DNA haltige Phase wurde nach der Zentrifugation bei 6 000 UpM in einer Tischzentrifuge der Firma Heraeus zweimal mit Ether extrahiert um Phenolreste zu beseitigen. Nach Zugabe von Isopropanol wurde die ausgefällte DNA bei 5 000 UpM abzentrifugiert und das Pellet mit 70 %igem Ethanol gewaschen. Der DNA-Niederschlag wurde in einer Speedvac-Zentrifuge getrocknet und dann in 1-2 ml Puffer gelöst.

Zum Nachweis der integrierten PRV-DNA wurden PRV-Primer aus dem 5' und 3' terminalen Bereich des 180 bp PRV-Fragmentes (SPN 345.1) eingesetzt um nach der PCR-Methode eine spezifische Amplifikation der integrierten PRV-DNA zu erreichen. Zur PCR-Reaktion wurden eingesetzt 2 $\mu$g genomische DNA aus den Veroklonen, 2 $\mu$mol Primer 1 und Primer 2, 2,5 Units Taq-Polymerase von Cetus/Perkin-Elmer sowie jeweils 200 $\mu$mol dNTPS in insgesamt 100 $\mu$l PCR-Puffer (50 nM KCl, 10 nM Tris/HCl pH 8,3, 1,5 nM $MgCl_2$ und 0,01 % Gelatine. Die Amplifikation wurde in einem PCR-Prozessor der Firma Cetus/Perkin-Elmer durchgeführt.

Mit den Ansätzen wurde zunächst eine Initialschmelzung der DNA 2 Minuten, 30 Sek. bei 94°C durchgeführt, dann pro Zyklus 1 Min. bei 94°C die DNA denaturiert, 2 Minuten bei 40-45°C das Primer-Annealing und 3 Minuten bei 72°C die Primer-Extension durchgeführt. Nach 35 Zyklen wurde abschließend eine 20 Minuten-Extension bei 72°C durchgeführt und die Ansätze bei 4°C gekühlt.

Die amplifizierten DNA-Proben wurden durch 1 %ige Agarosegelelektrophorese aufgetrennt und die denaturierte DNA nach der Southern Blot Hybridisierungstechnik auf eine Nitrozellulosemembran BA 85 der Firma Schleicher und Schüll übertragen und bei 80°C im Vakuumofen fixiert.

Die Nitrozellulosemembran wurde in einer Plastik-Folie verschweißt und die Hybridisierung nach Standardmethoden (Maniatis) durchgeführt.

Als Gensonden wurde Digoxigenin-dUTP markiertes oder $P^{32}$-dCTP markiertes 180 bp PRV-gII-Fragment (SPN 345.1) eingesetzt.

Die Auswertung der Southern Blots erfolgt mit Digoxigenin-Antikörpern, die mit alk. Phosphatase konjugiert waren und Nitroblautetrazolin/Bromochloroindolylphosphat nach der Methode der Fa. Boehringer bzw. durch Autoradiographie.

Die Southern Blot Hybridisierung mit der PCR amplifizierten genomischen DNA ergab, daß alle amplifizierten DNA-Fragmente mit einer Länge von ca. 0,2 kb ganz spezifisch mit der 180 bp Gensonde von gII-PRV hybridisierten, während die nicht transfizierte Verozell-DNA kein Hybridisierungssignal ergab.

Damit konnte eindeutig gezeigt werden, daß die Antisense-PRV-DNA im Genom der PRV resistenten Veroklone vorhanden ist. In Abb. 4 ist die Southern Blot/PCR Hybridisierung mit der 180 bp-PRV-Gensonde dargestellt.

Primer 1 ist 5'd(TCACGGACGTGATCGACC)3'

Primer 2 ist 5'd(TGTAGGTGTCGTTGGTGGT)3'

dNTPS sind Desoxinucleosidtriphosphate

## Beispiel 4

**Nachweis der Visrusresistenz von transfizierten, klonierten Verozellinien**

Klonierte Zellinien, die entsprechend der Beispiele 1,2 und 3 erhalten und charakterisiert wurden, wurden in vergleichenden Infektionsstudien mit PRV zusammen mit der Ausgangszellinie Vero als Kontrolle auf die Ausprägung der Virusresistenz untersucht.

**Infektion von Verozellen und PRV-Antisense-RNA enthaltende Veroklone mit PRV**

**Virustitration zur Bestimmung der kulturinfektiösen Dosis (KiD$_{50}$)**

Als Nährlösung für die Anzucht der Veroklone und Vero-Wildtyp-Zellen wurde Medium 199 der Fa. Flow mit den im Beispiel 2 beschriebenen Zusätzen benutzt. Es wurden 100 000 Zellen/ml Medium vom Wildtyp und den zu testenden Veroklonen ausgesät und 2-3 Tage bei 37°C inkubiert Danach wurde das Medium abgesaugt und durch Viruswachstumsmedium (MEME, Fa. Flow + 2 % FCS) ersetzt. Die Zellen wurden mit einer Virusverdünnung von 2x $10^{-3}$ bis 1x $10^{-7}$ der Originalvirusanzucht infiziert und bei 37°C inkubiert Nach ca. 18-20 Stunden wurde die Inkubation durch Einfrieren bei -80°C abgebrochen. Zur Bestimmung des KiD$_{50}$ wurden BK-Zellen zu 200 000 Zellen pro ml Nährlösung in 96 Wells-Mikrotiterplatten ausgesät und über Nacht bei 37°C im $CO_2$-Brutrchrank inkubiert Das Medium war MEME + 10 % FCS. MEME ist Minimum Essential Medium Eagle mit Salzen in mg/Liter: L-Arginin 126,4; L-Cystin 28,4; L-Glutamin 292,3; L-Histidin 41,9; L-Isoleucin 52,5; L-Leucin 52,5; L-Lysin 73,06; L-Methionin 14,9; L-Phenylalanin 33,02; L-Threonin 47,64; L-Tryptophan 10,2; L-Tyrosin 45,02; L-Valin 46,9; D-Ca-Pantothenat 1; Cholinchlorid 1; Folsäure 1; Inosit 2; Nicotinamid 1; Pyridoxal/HCl 1; Riboflavin 0,1; Thiamin 1,0, $CaCl_2 \cdot 2H_2O$ 264,9; KCl 400; $MgSO_4 \cdot 7H_2O$ 200; NaCl 6800; $NaHCO_3$ 2000; $NaH_2PO_4 \cdot 2H_2O$ 158,3; D-Glucose 1000; Phenolrot 17;

Die bei -80°C eingefrorenen Viruskulturen wurden aufgetaut und die Viruskultur von $10^{-1}$ bis $10^{-8}$ in MEME + 3 % FCS verdünnt und von den Mikrotiterplatten mit den BK-Zellen wurde das Medium abgesaugt und die Virusverdünnungen auf die BK-Zellen gegeben. Nach 6 Tagen Inkubation im $CO_2$-Brutrchrank bei 37°C wurde der cytopathologische Effekt (CPE) mikroskopisch abgelesen.

Mikrotiter-Wells, in denen Plaques auftraten, wurden als positiv gewertet.

**Plaquetest: Bestimmung der infektiösen Einheiten (IU)**

Wildtypzellen von Vero-Wildtypzellen und Veroklonen mit integrierten PRV-Antisense-Konstrukten wurden zu 100 000 Zellen pro ml in Medium 199 + 5 % FCS ausgesät und 2-3 Tage bei 37°C inkubiert.

Anschließend wurde das Medium gegen MEME-Medium + 2 % FCS ausgetauscht und Virus in den Verdünnungen $10^{-7}$ und $10^{-8}$ zugesetzt. Nach 60-70 Stunden wurde das Wachstum der Viren durch Einfrieren bei -80°C gestoppt. Zur Bestimmung der infektiösen Einheiten wurden Gewebekulturplatten (Ø 5 cm) mit Vero-Wildtyp-Zellen angelegt. Vero-Wildtyp wurde dazu in Medium 199 + 5 % FCS über Nacht inkubiert und anschließend mit den Virusinkubationen vom Wildtyp und den Veroklonen in Vedünnungen von $10^{-1}$ und $10^{-7}$ infiziert. Nach 3 Stunden Inkubation im $CO_2$-Brutrchrank wurde Methycellulose-Medium (MEME + 5 % FCS + 3,75 g Methylcellulose auf 500 ml) zugesetzt und so lange weiter inkubiert bis Plaques im Zellktulturrasen erkennbar waren.

Anschließend wurde mit einer 5 %igen Formaldehydlösung fixiert und mit 20 %iger Giemsalösung angefärbt. Die Plaques wurden ausgezählt, die Aazahl der Plaques multipliziert mit der Verdünnung ergaben die infektiösen Einheiten (IU).

Im Rahmen dieser Tests wurde festgestellt, daß die in der Abbildung Fig. 4 genannten Veroklone VK18, VK44, VK66, VK69, VK120 und VK159, die Antisense-Konstrukte mit dem 180 bp langen PRV-Fragment aus der gII-Region enthalten, gegen PRV resistent sind, während die Vero-Wildtyp-Zellen und Klone mit integrierten Expressionsvektoren ohne PRV-Insert normale Virussensitivität besitzen.

## Beispiel 5

### Nachweis der PRV Antisense-RNA-Expression in Verozellen

Die Antisense-RNA-Expression in den PRV resistenten Veroklonen wurde mit der Northern Blot Technik überprüft. Die Isolierung der zellulären RNA wurde mit einer Guanidin Isothiocyanat-Standardmethode durchgeführt.

Als Puffer wurden verwendet: GII-Puffer (94,53 g Guanidinthioisocyanat, 1,67 ml 3M Natriumacetat pH 6 mit $H_2O$ auf 200 ml auffüllen + 1,67 ml ß-Mercaptoethol) und CsCl-Puffer ( 95,97 ml CsCl + 0,83 ml 3M Natriumacetat pH 6 mit $H_2O$ auf 100 ml auffüllen, sterilfiltrieren).

Die Zellen wurden in 8 ml GII-Puffer aufgetaut und vorsichtig geschüttelt bis sie vollständig lysiert waren. In SW40 Zentrifugenröhrchen wurden 4 ml CsCl-Puffer pipettiert und dann vorsichtig mit dem Zellysat überschichtet. Nach 12 Stunden Zentrifugation im SW40 Rotor bei 32 000 UpM wurden die einzelnen Phasen abpipettiert, aus den Röhrchen die Restflüssigkeit abgetupft und dann der RNA-Niederschlag am Boden des Rötuchens in 2 X 200 $\mu$l 0,3 M Natriumacetat pH 6 aufgenommen und vorsichtig resuspendiert. Die RNA wurde anschließend mit 2,5 Volumina Ethanol versetzt und 30 Minuten bei -70°C ausgefällt, 10 Minuten bei 5 000 UpM in der Tischzentrifuge abzentrifugiert und das RNA-Pellet in 200 $\mu$l 70 %igem Ethanol aufgenommen und bei -20°C gelagert. Die Gelelektrophorese der RNA und das Northern Blotting wurde wie nachstehend beschrieben durchgeführt.

Für die Elektrophorese von RNA und Northern Blotting wurden folgende Reagenzien und Puffer verwendet:

| | |
|---|---|
| Reagenzien: | 10x Mopspuffer (0,2M 3-morpholinopropanesulfonicacid 0,05 M Natriumacetat 0,01 M EDTA pH 5,5-7,0) |
| | 37% Formaldehydlösung |
| Ladepuffer: | 0,72 ml Formamid |
| | 0,16 ml 10x-Mops |
| | 0,26 ml Formaldehyd 37 % |
| | 0,18 mi bidest•$H_2O$ |
| | 0,10 ml 80% Glycerin |
| | 0,08 ml Bromphenolblau gesättigt in bidest•$H_2O$ |
| Transfer: | Steriles 10xSSC (1,5 M NaCl; 0,15 M Nacitrat) |
| Agarosegel: | 1 g Agarose |
| | 10 ml 10x Mops |
| | 85 ml bidest•$H_2O$ |
| | in Microwelle lösen, auf 50° abkühlen |
| | + 5,5 mi 37 % Formaldehydlösung |

Die Gelelektrophorese wurde in 1x Mops bei 193V,55 mA ca. 1,5 Stunden durchgeführt.

Zur Denaturietung der RNA wurden die Ethanol gefällten Proben bei 5000 UpM zentrifugiert. Der Überstand wurde abgegossen und der Niederschlag ca. 30 Minuten bei Raumtemperatur getrocknet. Zu den Proben wurden 20 $\mu$l Ladepuffer zupipettiert und 2 Minuten bei 95°C denaturiert und auf 4°C abgekühlt. Zu den Proben wurden 0,5 $\mu$l Ethidiumbromid (10 mg/ml) zupipettiert und die Ansätze auf das Gel aufgetragen. Es wurden mindestens 10-15 $\mu$g Gesamt-RNA verwendet.

Zum Blotten wurde das Gel zunächst 2x 15 Minuten in 10xSSC äquilibriert um das Formaldehyd zu entfernen. Der Transfer der RNA auf vorbehandelte Nitrozellulosemembran (1 Min bidest $H_2O$ äquitibriert mit 10xSSC) ertolgte analog wie beim Southern Transfer.

Zur Hybridisierung nach dem Northern Transfer wurde die Nitrozellulosemembran in 30-40 ml Prähybridisierungspuffer (50 % Formamid, 5xSSC, 50 mM Natriumphosphat pH 6.5, 2,0 $\mu$g/ml ultrabeschallte Heringsperma DNA, 10x Denhardlösung) 8-20 Stunden bei 42°C behandelt. Der Prähybridisierungspuffer wurde verworfen und durch Hybridisierungspuffer (4 Teile Prähybridisierungspuffer, 1 Teil 50%ige Dextransulfatlösung) ersetzt. Die Gensonde (10 ng/ml) spez. Aktivität $10^8$ cpm/$\mu$g) wurde zugegeben und der Plastikbeutel mit der Nitrozellulosemembran wieder versiegelt und unter leichtem Schütteln 20 Stunden bei 42°C hybridisiert.

Die Membran wurde anschließend 4 mal mit 2xSSC + 0,1 % SDS 15 Minuten bei 50°C gewaschen, dann in Plastikfolie verpackt und mit Röntgenfilm unter Verwendung einer Enhancement-Folie exponiert.

In Northern Blots von zellulärer RNA aus den virusresistenten Veroklonen VK13 und VK18 konnte beispielhaft gezeigt werden, daß Antisense-RNA-Transkripte vorhanden sind, die ganz spezifisch mit der 180 bp Gensonde aus SPN 345.1 hybridisierten. In zellulärer RNA von nicht transfizierten Verozellen wurden dagegen keine Antisense-Transkripte nachgewiesen.

## Beispiel 6

**Nachweis der PRV Resistenz in Verozellen, die mit Antisense-Oligonukleotiden vorbehandelt wurden**

Die biologische Wirksamkeit von Antisense-Oligonukleotiden der folgenden Sequenzprotokolle (Sequence Listing 28769/1-17), die spezifisch auf die RNA des gII-Gens von PRV einwirken, wurde durch einen Virusresistenztest gemessen. Die Verozellen wurden 1-3 Tage vorbehandelt in Gegenwart von verschiedenen Konzentrationen (10 $\mu$g-100 $\mu$g/ml) von Antisense-Oligonukleotiden. Die PRV-Resistenz wurde wie im Beispiel 4 beschrieben gemessen.

SEQUENZPROTOKOLL


(1) ALGEMEINE INFORMATION:

   (i) ANMELDER:
     (A) NAME: BAYER AG
     (B) STRASSE: Bayerwerk
     (C) ORT: Leverkusen
     (E) LAND: Deutschland
     (F) POSTLEITZAHL: 5090
     (G) TELEPHON: 0214/30 66400
     (H) TELEFAX: 0214/30 3482
     (I) TELEX: 85 101-265 by d

  (ii) ANMELDETITEL: Pseudorabies-Virus (PRV)-Polynukleotide und
     ihre Verwendung zur Herstellung von virusresistenten
     eukaryotischen Zellen

  (iii) ANZAHL DER SEQUENZEN: 18

  (iv) COMPUTER-LESBARE FORM:
     (A) DATENTRÄGER: Floppy disk
     (B) COMPUTER: IBM PC compatible
     (C) BETRIEBSSYSTEM: PC-DOS/MS-DOS
     (D) SOFTWARE: PatentIn Release #1.0, Version #1.25 (EPA)


(2) INFORMATION ZU SEQ ID NO: 1:

  (i) SEQUENZ CHARAKTERISTIKA:
     (A) LÄNGE: 20 Basenpaare
     (B) ART: Nukleinsäure
     (C) STRANGFORM: Einzel
     (D) TOPOLOGIE: linear

  (ii) ART DES MOLEKÜLS: DNS (genomisch)


  (xi) SEQUENZBESCHREIBUNG: SEQ ID NO: 1:

CCAGCTAGTG CAGGCACTAG       20

(2) INFORMATION ZU SEQ ID NO: 2:

  (i) SEQUENZ CHARAKTERISTIKA:
     (A) LÄNGE: 20 Basenpaare
     (B) ART: Nukleinsäure
     (C) STRANGFORM: Einzel
     (D) TOPOLOGIE: linear

  (ii) ART DES MOLEKÜLS: DNS (genomisch)


  (xi) SEQUENZBESCHREIBUNG: SEQ ID NO: 2:

CCTCTGCGTG AACGGCGCCG       20

(2) INFORMATION ZU SEQ ID NO: 3:

  (i) SEQUENZ CHARAKTERISTIKA:
     (A) LÄNGE: 20 Basenpaare

            (B) ART: Nukleinsäure
            (C) STRANGFORM: Einzel
            (D) TOPOLOGIE: linear

        (ii) ART DES MOLEKÜLS: DNS (genomisch)


        (xi) SEQUENZBESCHREIBUNG: SEQ ID NO: 3:

AACGCGTGCA TGAGCCGGAA                                                    20

(2) INFORMATION ZU SEQ ID NO: 4:

        (i) SEQUENZ CHARAKTERISTIKA:
            (A) LÄNGE: 20 Basenpaare
            (B) ART: Nukleinsäure
            (C) STRANGFORM: Einzel
            (D) TOPOLOGIE: linear

        (ii) ART DES MOLEKÜLS: DNS (genomisch)


        (xi) SEQUENZBESCHREIBUNG: SEQ ID NO: 4:

TCCGCCAGTG GAACACCAAC                                                    20

(2) INFORMATION ZU SEQ ID NO: 5:

        (i) SEQUENZ CHARAKTERISTIKA:
            (A) LÄNGE: 20 Basenpaare
            (B) ART: Nukleinsäure
            (C) STRANGFORM: Einzel
            (D) TOPOLOGIE: linear

        (ii) ART DES MOLEKÜLS: DNS (genomisch)


        (xi) SEQUENZBESCHREIBUNG: SEQ ID NO: 5:

CCCGCGTCCA GGTGGAGCTG                                                    20

(2) INFORMATION ZU SEQ ID NO: 6:

        (i) SEQUENZ CHARAKTERISTIKA:
            (A) LÄNGE: 20 Basenpaare
            (B) ART: Nukleinsäure
            (C) STRANGFORM: Einzel
            (D) TOPOLOGIE: linear

        (ii) ART DES MOLEKÜLS: DNS (genomisch)


        (xi) SEQUENZBESCHREIBUNG: SEQ ID NO: 6:

GCTCGCGCAA GTCCGCGCTC                                                    20

(2) INFORMATION ZU SEQ ID NO: 7:

        (i) SEQUENZ CHARAKTERISTIKA:
            (A) LÄNGE: 20 Basenpaare

```
        (B)  ART: Nukleinsäure
        (C)  STRANGFORM: Einzel
        (D)  TOPOLOGIE: linear

    (ii)  ART DES MOLEKÜLS: DNS (genomisch)


    (xi)  SEQUENZBESCHREIBUNG: SEQ ID NO: 7:

CCACACGGTC GGCGCCCACG                                          20

(2)  INFORMATION ZU SEQ ID NO: 8:

    (i)  SEQUENZ CHARAKTERISTIKA:
        (A)  LÄNGE: 20 Basenpaare
        (B)  ART: Nukleinsäure
        (C)  STRANGFORM: Einzel
        (D)  TOPOLOGIE: linear

    (ii)  ART DES MOLEKÜLS: DNS (genomisch)


    (xi)  SEQUENZBESCHREIBUNG: SEQ ID NO: 8:

CCCCAAGAGC AGCGCCAGCT                                          20

(2)  INFORMATION ZU SEQ ID NO: 9:

    (i)  SEQUENZ CHARAKTERISTIKA:
        (A)  LÄNGE: 20 Basenpaare
        (B)  ART: Nukleinsäure
        (C)  STRANGFORM: Einzel
        (D)  TOPOLOGIE: linear

    (ii)  ART DES MOLEKÜLS: DNS (genomisch)


    (xi)  SEQUENZBESCHREIBUNG: SEQ ID NO: 9:

AACCACATCC ACAGCAACCA                                          20

(2)  INFORMATION ZU SEQ ID NO: 10:

    (i)  SEQUENZ CHARAKTERISTIKA:
        (A)  LÄNGE: 20 Basenpaare
        (B)  ART: Nukleinsäure
        (C)  STRANGFORM: Einzel
        (D)  TOPOLOGIE: linear

    (ii)  ART DES MOLEKÜLS: DNS (genomisch)


    (xi)  SEQUENZBESCHREIBUNG: SEQ ID NO: 10:

AACGGCGCCG CCAGCTAGTG                                          20

(2)  INFORMATION ZU SEQ ID NO: 11:

    (i)  SEQUENZ CHARAKTERISTIKA:
        (A)  LÄNGE: 20 Basenpaare
```

```
        (B)  ART: Nukleinsäure
        (C)  STRANGFORM: Einzel
        (D)  TOPOLOGIE: linear

    (ii)  ART DES MOLEKÜLS: DNS (genomisch)


    (xi)  SEQUENZBESCHREIBUNG: SEQ ID NO: 11:

TGAGCCGGAA CCTCTGCGTG                                                    20

(2)  INFORMATION ZU SEQ ID NO: 12:

    (i)  SEQUENZ CHARAKTERISTIKA:
        (A)  LÄNGE: 20 Basenpaare
        (B)  ART: Nukleinsäure
        (C)  STRANGFORM: Einzel
        (D)  TOPOLOGIE: linear

    (ii)  ART DES MOLEKÜLS: DNS (genomisch)



    (xi)  SEQUENZBESCHREIBUNG: SEQ ID NO: 12:

GAACACCAAC AACGCGTGCA                                                    20

(2)  INFORMATION ZU SEQ ID NO: 13:

    (i)  SEQUENZ CHARAKTERISTIKA:
        (A)  LÄNGE: 20 Basenpaare
        (B)  ART: Nukleinsäure
        (C)  STRANGFORM: Einzel
        (D)  TOPOLOGIE: linear

    (ii)  ART DES MOLEKÜLS: DNS (genomisch)



    (xi)  SEQUENZBESCHREIBUNG: SEQ ID NO: 13:

AGCGCCAGCT TCCGCCAGTG                                                    20

(2)  INFORMATION ZU SEQ ID NO: 14:

    (i)  SEQUENZ CHARAKTERISTIKA:
        (A)  LÄNGE: 20 Basenpaare
        (B)  ART: Nukleinsäure
        (C)  STRANGFORM: Einzel
        (D)  TOPOLOGIE: linear

    (ii)  ART DES MOLEKÜLS: DNS (genomisch)



    (xi)  SEQUENZBESCHREIBUNG: SEQ ID NO: 14:

GGTGGAGCTG CCCCAAGAGC                                                    20

(2)  INFORMATION ZU SEQ ID NO: 15:

    (i)  SEQUENZ CHARAKTERISTIKA:
        (A)  LÄNGE: 20 Basenpaare
```

17

        (B) ART: Nukleinsäure
        (C) STRANGFORM: Einzel
        (D) TOPOLOGIE: linear

    (ii) ART DES MOLEKÜLS: DNS (genomisch)


    (xi) SEQUENZBESCHREIBUNG: SEQ ID NO: 15:

GTCCGCGCTC CCCGCGTCCA                                                    20

(2) INFORMATION ZU SEQ ID NO: 16:

        (i) SEQUENZ CHARAKTERISTIKA:
            (A) LÄNGE: 20 Basenpaare
            (B) ART: Nukleinsäure
            (C) STRANGFORM: Einzel
            (D) TOPOLOGIE: linear

    (ii) ART DES MOLEKÜLS: DNS (genomisch)



    (xi) SEQUENZBESCHREIBUNG: SEQ ID NO: 16:

GGCGCCCACG GCTCGCGCAA                                                    20

(2) INFORMATION ZU SEQ ID NO: 17:

        (i) SEQUENZ CHARAKTERISTIKA:
            (A) LÄNGE: 20 Basenpaare
            (B) ART: Nukleinsäure
            (C) STRANGFORM: Einzel
            (D) TOPOLOGIE: linear

    (ii) ART DES MOLEKÜLS: DNS (genomisch)



    (xi) SEQUENZBESCHREIBUNG: SEQ ID NO: 17:

ACAGCAACCA CCACACGGTC                                                    20

(2) INFORMATION ZU SEQ ID NO: 18:

        (i) SEQUENZ CHARAKTERISTIKA:
            (A) LÄNGE: 381 Basenpaare
            (B) ART: Nukleinsäure
            (C) STRANGFORM: Doppel
            (D) TOPOLOGIE: linear

    (ii) ART DES MOLEKÜLS: DNS (genomisch)

    (iii) ANTISENSE: JA


    (xi) SEQUENZBESCHREIBUNG: SEQ ID NO: 18:

GATCACGGAC GTGATCGACC GCCGCGGCAA GTGCGTCTCC AAGGCCGAGT CTAGTGCCTG    60

CACTAGCTGG CGGCGCCGTT CACGCAGAGG TTCCGGCTCA ACGTGCGCAA CAACCACAAG   120


18

```
GTGACCGCCT  TCGACCGCGA  CGAGAACCCC  TGCACGCGTT  GTTGGTGTTC  CACTGGCGGA      180

AGCTGGCGCT  GCTCTTGGGG  GTCGAGGTGG  ACCTGCGCCC  CTCGCGCCTG  AACGCGCTCG      240

GCACCCGCGG  CAGCTCCACC  TGGACGCGGG  GAGCGCGGAC  TTGCGCGAGC  CGTGGGCGCC      300

CTGGCACACC  ACCAACGACA  CCTACACCAA  GACCGTGTGG  TGGTTGCTGT  GGATGTGGTT      360

GCGTATCCTT  ATGATCGCAA  T                                                  381
```

## Beschreibungen der Abbildungen

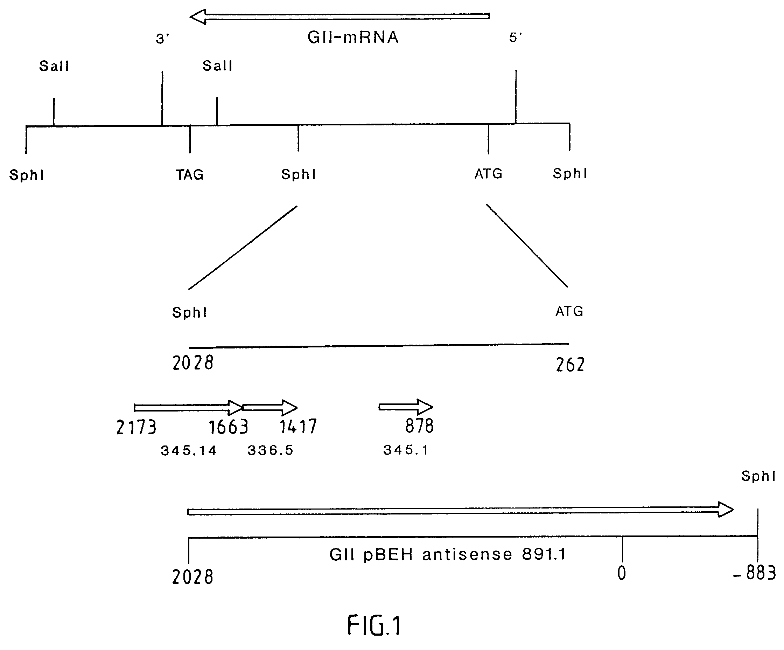

Abbildung Fig. 1 zeigt einen Ausschnitt aus der Restriktionskarte von PRV mit der gII-Region von PRV und der Lokalisierung von beispielhaft aufgeführten Antisensekonstrukten.

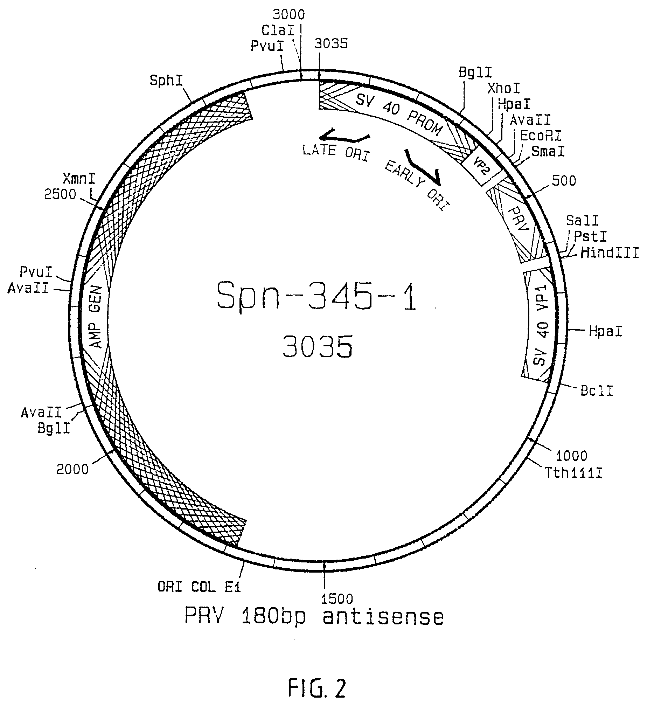

Abbildung Fig. 2 zeigt die Vektorkarte von pBEH mit dem 180 bp langen PRV gII-Fragment (Sequenzbereich 879-1058) in Antisense-Orientierung zu dem SV40 early Promotor des Vektors.

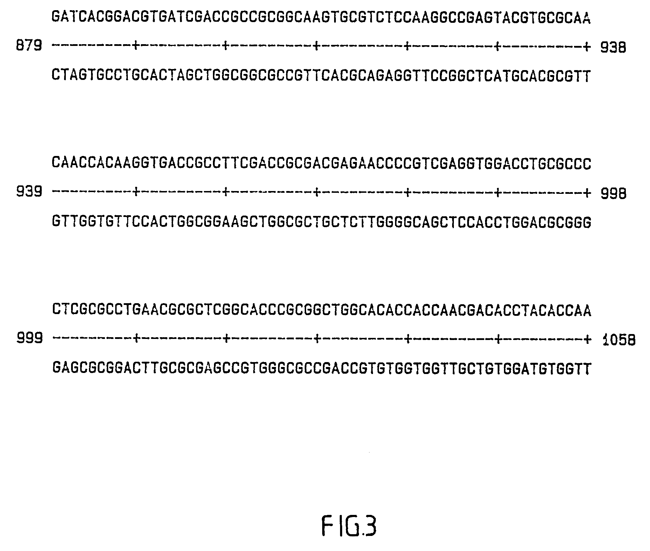

Abbildung Fig. 3 zeigt die Nukleotidsequenz des 180 bp langen DNA-Fragmentes aus der gII-Region von PRV (Sequenzbereich 879-1058), das in Antisense-Orientierung zu einem Promotor eines Eukaryonten-Expressionsvektors eine hohe Resistenz gegen PRV in Eukaryontenzellen erzeugt.

Abbildung 4 zeigt einen Southern Blot von genomischer DNA aus den mit SPN 345.1 Antisense-Konstrukt transfizierten Veroklonen VK18, 44, 66, 69, 120, 159 sowie den nicht transfizierten Verozellinien Vero 177 und 210. Als Positivkontrollen wurde das 180 bp-Fragment 345.1 sowie das Plasmid 345.1 mit dem 180 bp-Insert eingesetzt. Als Negativkontrolle wurde ein 250 bp-Fragment (1417-1663) aus der gII-Region verwendet.

## Patentansprüche

1. Antisense DNA Polynukleotide, die dadurch gekennzeichnet sind, daß sie Gensequenzen von PRV enthalten, die in Antisenseorientierung zu einer Promotorsequenz lokalisiert sind.

2. Antisense DNA Polynukleotide, die dadurch gekennzeichnet sind, daß sie Gensequenzen aus der gII-Region von PRV enthalten, die in Antisenseorientierung zu einer Promotorsequenz lokalisiert sind.

3. Antisense DNA Polynukleotide, die dadurch gekennzeichnet sind, daß sie eine 180 bp große Region entsprechend der Nukleotidsequenz: 879-1058 der gII-Region von PRV enthalten, die in Antisenseorientierung zu einer Promotorsequenz lokalisiert ist.

4. Antisense RNA Polynukleotide, dadurch gekennzeichnet, daß sie Gensequenzen von PRV enthalten.

5. Antisense RNA Polynukleotide, dadurch gekennzeichnet, daß sie Gensequenzen aus der gII-Region von PRV enthalten.

6. Antisense RNA Polynukleotide, dadurch gekennzeichnet, daß sie eine 180 bp große Region entsprechend der Nukleotidsequenz 879-1058 der gII-Region von PRV enthalten.

7. Antisense Oligonukleotide, dadurch gekennzeichnet, daß sie Gensequenzen von PRV enthalten, die komplementär zu viralen RNA's von PRV sind.

8. Antisense Oligonukleotide, dadurch gekennzeichnet, daß sie Gensequenzen von der gII-Region von PRV enthalten, die komplementär zur viralen gII RNA von PRV sind.

9. Antisense Oligonukleotide, dadurch gekennzeichnet, daß sie Gensequenzen aus einer 180 bp großen Region (879-1058) aus dem gII-Bereich von PRV enthalten, die komplementär zur viralen gII RNA sind.

10. Expressionsvektorkonstrukte enthaltend Polynukleotide aus PRV in Antisense-Orientierung zu Promotorsequenzen.

11. Expressionsvektorkonstrukte enthaltend Polynukleotide aus der gII-Region von PRV in Antisense-Orientierung zu Promotorsequenzen.

12. Expressionsvektorkonstrukte enthaltend ein 180 bp Polynukleotid (879-1058) aus der gII-Region von PRV in Antisense-Orientierung zu Promotorsequenzen.

13. Zellen und Nachkommen dieser Zellen enthaltend Antisense Polynukleotide nach Punkten 1 bis 6.

14. Schweine, die in ihren Zellen Antisense-Polynukleotide nach Ansprüchen 1 bis 6 und 10 bis 12 enthalten.

15. Schweine, die in ihren Zellen Antisense-Oligonukleotide nach Ansprüchen 7 bis 9 enthalten.

16. Verfahren zur Herstellung von PRV resistenten Zellen, dadurch gekennzeichnet, daß Polynukleotide nach Ansprüchen 1 bis 6 oder 10 bis 12 in Virus sensitive Zellen übertragen werden und RNA komplementär zur viralen RNA gebildet wird und dadurch die virale Vermehrung von PRV verhindert wird.

17. Verfahren zur Hemmung von PRV-Infektionen, dadurch gekennzeichnet, daß Antisense-Oligonukleotide nach Anspruch 7 bis 9 in Tiere appliziert werden.

18. Verwendung von Polynukleotide gemäß Ansprüchen 1 bis 6 oder 10 bis 12 zur Übertragung in Zellen, die zur Erzeugung von transgenen PRV-resistenten Tieren geeignet sind.

FIG.1

FIG. 2

GATCACGGACGTGATCGACCGCCGCGGCAAGTGCGTCTCCAAGGCCGAGTACGTGCGCAA

879 ---------+---------+---------+---------+---------+---------+ 938

CTAGTGCCTGCACTAGCTGGCGGCGCCGTTCACGCAGAGGTTCCGGCTCATGCACGCGTT


CAACCACAAGGTGACCGCCTTCGACCGCGACGAGAACCCCGTCGAGGTGGACCTGCGCCC

939 ---------+---------+---------+---------+---------+---------+ 998

GTTGGTGTTCCACTGGCGGAAGCTGGCGCTGCTCTTGGGGCAGCTCCACCTGGACGCGGG


CTCGCGCCTGAACGCGCTCGGCACCCGCGGCTGGCACACCACCAACGACACCTACACCAA

999 ---------+---------+---------+---------+---------+---------+ 1058

GAGCGCGGACTTGCGCGAGCCGTGGGCGCCGACCGTGTGGTGGTTGCTGTGGATGTGGTT


# FIG.3

FIG.4